# EUROPEAN PATENT SPECIFICATION

(11) **EP 3 169 302 B1**
(45) Date of publication and mention of the grant of the patent: **27.02.2019**
(21) Application number: 15821858.6
(22) Date of filing: 15.07.2015
(51) Int. Cl.: A61J 9/00, A61J 15/00

(54) **BABY FEEDING KIT**
KIT FÜR BABYFÜTTERUNG
TROUSSE D'ALIMENTATION POUR BÉBÉ

(30) Priority: 15.07.2014 US 201462024474 P
(43) Date of publication of application: 24.05.2017
(73) Proprietor: Bezalel Laboratories Ltd., 9124001 Jerusalem (IL)
(72) Inventor: KORIAT-BARKAN, Ravid, 6802817 Tel Aviv (IL)
(74) Representative: Jakelski & Althoff Patentanwälte PartG mbB
(86) International application number: PCT/IL2015/050734
(87) International publication number: WO 2016/009437

(56) References cited:
- WO-A1-2014/143130
- CN-A- 102 920 609
- GB-A- 2 429 169
- US-A- 3 645 262
- US-B2- 7 799 008

## Description

### FIELD OF THE INVENTION

The present invention relates to baby feeding containers in general, and to a container for feeding premature babies in particular. Such a device is disclosed in the US3645262.

### BACKGROUND OF THE INVENTION

Premature babies or preemies are human children that are born after less than 37 weeks of pregnancy, which is more than three weeks before their due date. Premature birth gives the baby less time to develop in the womb, and as a result a preemie's body is often vulnerable, weak, and low in weight. Premature babies, especially those born very early, often have complicated medical problems and require neonatal intensive care. Providing neonatal intensive care to preemies can be a difficult and stressful task for the medical staff charged with providing the care, the families of the babies, and the preemies themselves. The length of stay for a preemie in the Neonatal Intensive Care Unit (NICU) in a Hospital can last a relatively long time, until the preemie's physical condition is strong and stable enough for him to be discharged.

One of the critical and frequent occurrences for a preemie in the NICU is feeding time. Breast feeding requires a relatively large amount of physical strength and effort on the part of the preemie, and can be a complex process for a preemie due to his size and capabilities. Accordingly, breast milk, which is favored by doctors, cannot be naturally fed directly from the mother's breast. Instead, breast milk is transmitted from the mother's breast to the preemie using several containers, tools and vessels. Typically a mother will express milk using a pump into a vessel. The milk is then transferred from that vessel into a storage container that is marked and placed in cold storage. During feeding time the storage container is removed from storage and a syringe is used to measure an amount of milk to be fed to the preemie. This milk is then transferred to a bottle and fed to the preemie. Since it is critical for the preemie to feed properly, any milk that remains in the bottle after bottle feeding is given to the preemie directly. The milk is therefore transferred, once again, this time from the bottle to a feed-tube that inputs the milk into the preemie's stomach. Each time the milk is transferred to a different container complicates the feeding process, and more importantly exposes it to possible contamination and loss of nutritional value.

### SUMMARY OF THE INVENTION

In accordance with one aspect of the present invention, there is thus provided a baby feeding kit, wherein the kit is operational in syringe mode, storage mode, feed-bottle mode, and feed-tube mode, as further disclosed in claim 1. The kit includes a container body configured for holding a liquid. The container body includes a tube portion defining a cavity therein, a wide opening disposed at one end of the tube portion, and a narrow opening disposed at an opposite end of the tube portion. The kit further includes a plunger sized to removably and sealingly fit inside the cavity and slidably move there along when the container is in a syringe mode, and to seal the tube portion when the container is in a storage mode. The kit further includes an attachable nipple operational for sealingly attaching to the wide opening, configured for releasing a liquid held in the container body when sucked on when the container is in a feed-bottle mode in which the plunger is removed, and for pressurizing fluids when the container is in a feed-tube mode, and which is removed when the container is in a syringe or storage mode. The kit further includes an attachable cap operational for sealingly attaching to the narrow opening, and is configured to seal the narrow opening of the container when in storage or in feed-bottle mode. The kit further includes a feeding tube operational for sealingly attaching to the narrow opening, and is configured for releasing a liquid held in the container body when the container is in a feed-tube mode.

In accordance with another aspect of the present invention, at least one of the wide opening and the narrow opening includes an attachment element that corresponds to a complementary attaching element disposed on at least one of: the nipple, the cap, and the feeding tube for attaching thereto.

In accordance with another aspect of the present invention the attachment element and the complementary attachment element are screw threads.

In accordance with another aspect of the present invention the kit further includes a gripping sleeve configured to slide externally onto and off of the container body, and to provide a grip support facilitating holding of the container body in the feed bottle mode and optionally having an securing element, such as a clip, for securing the gripping sleeve to an object.

The baby feeding kit of claim 4, further comprising a carrier for removably storing and carrying the gripping sleeve. The carrier can include a handle for providing a hanging suspension for the gripping sleeve, and a hanger for hanging the carrier.

In accordance with another aspect of the present invention the kit further includes a carrier, wherein

the gripping sleeve is configured to be removably stored inside the carrier.

In accordance with another aspect of the present invention the kit is operational in a pump vessel mode. The wide opening is configured for attaching to a breast pump, which can be part of the kit, for pumping milk from a breast into the container body.

### BRIEF DESCRIPTION OF THE DRAWINGS

The present invention will be understood and appreciated more fully from the following detailed description taken in conjunction with the drawings in which:
Figure 1 is an exploded plan view of a container, constructed and operative in accordance with an embodiment of the present invention;
Figure 2 is a perspective view of the container of Figure 1 in a syringe mode;
Figure 3 is a perspective view of the container of Figure 1 in a storage container mode;
Figure 4 is a perspective view of the container of Figure 1 in a feed-bottle mode;
Figure 5 is a perspective view of the container of Figure 1 in a feed-tube mode;
Figure 6 is a perspective view of the sleeve and carrier of Figure 1;
Figure 7 is a perspective view of the container of Figure 1 in a feed-tube mode and being held by the sleeve and carrier; and
Figure 8 is a perspective view of the container of Figure 1 in a pump vessel mode.

### DETAILED DESCRIPTION OF THE EMBODIMENTS

The present invention overcomes the disadvantages of the prior art by providing a kit with a container that simplifies the feeding process for preemies. The container can be adapted for use as a syringe, storage container, feed-bottle or feeding tube. Optionally, the container can be used as a pump vessel, connecting directly to a breast pump. The container can be used as a syringe to measure the exact amount of milk given to the preemie, a critical step in the feeding process for preemies that need to feed properly to develop properly. The term "milk" herein refers to any liquid, including artificial substitutes, water, medical preparations, and the like, which is intended for feeding. The container can be sealed for further storage. The container is readily adapted for use as a feed-bottle which allows a parent or caregiver to feed the preemie without having to transfer the milk. Additionally, the container can be adapted for use as a feeding tube to input the milk into the stomach of the preemie. The milk remains in the container from its filling (syringe phase or as a pump vessel) until its consumption by any of the modes (syringe, storage, feed-bottle, feeding tube), without requiring its transfer into further vessels.

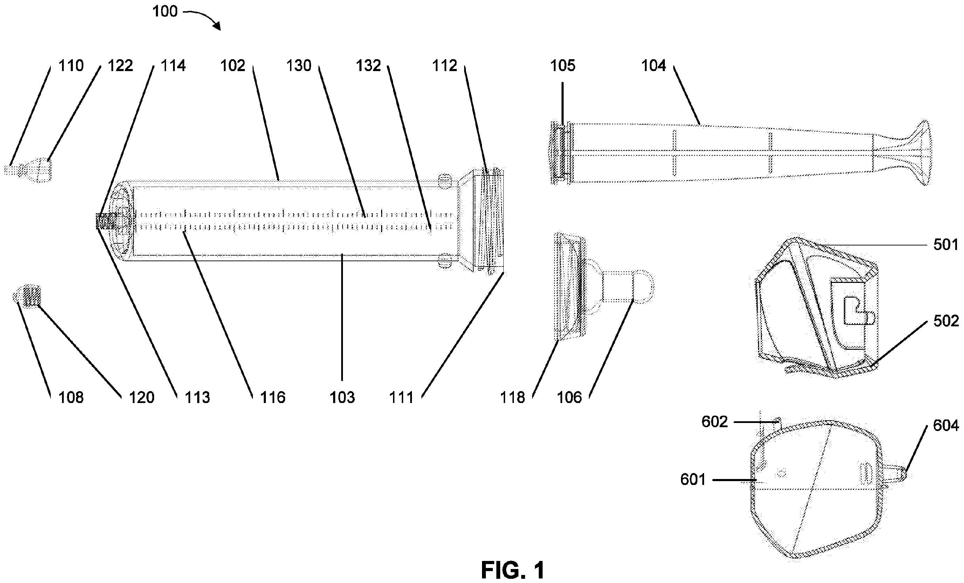

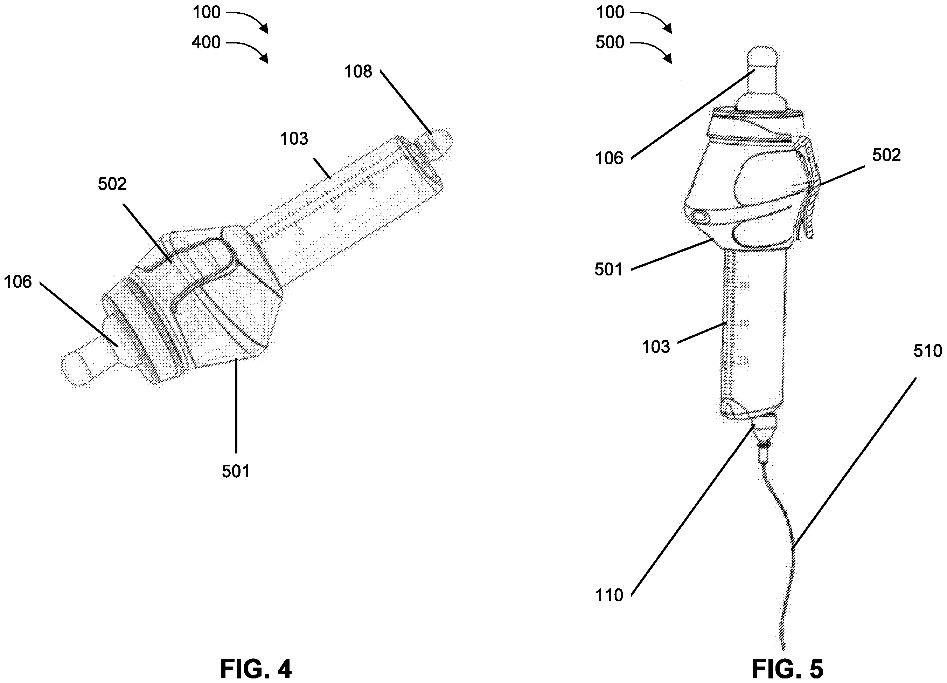

Reference is now made to Figure 1, which is an exploded plan view of a container kit 100, constructed and operative in accordance with an embodiment of the present invention. The invention present concerns a kit, however, add-on elements may be utilized in conjunction with existing elements to receive the kit. Accordingly reference herein below to "container 100" is made for convenience purposes and is synonymous to "kit 100". Container 100 includes a body 102, a plunger 104, a nipple 106, a cap 108, a feeding tube 110, an optional gripping sleeve 501, and an optional carrier 601. Feeding tube 110 incorporates an adapter portion (designated 110 for the sake of simplicity) as in Figures 1, 5 and 7, while a tube portion 510 appears hanging therefrom in Figure 5.

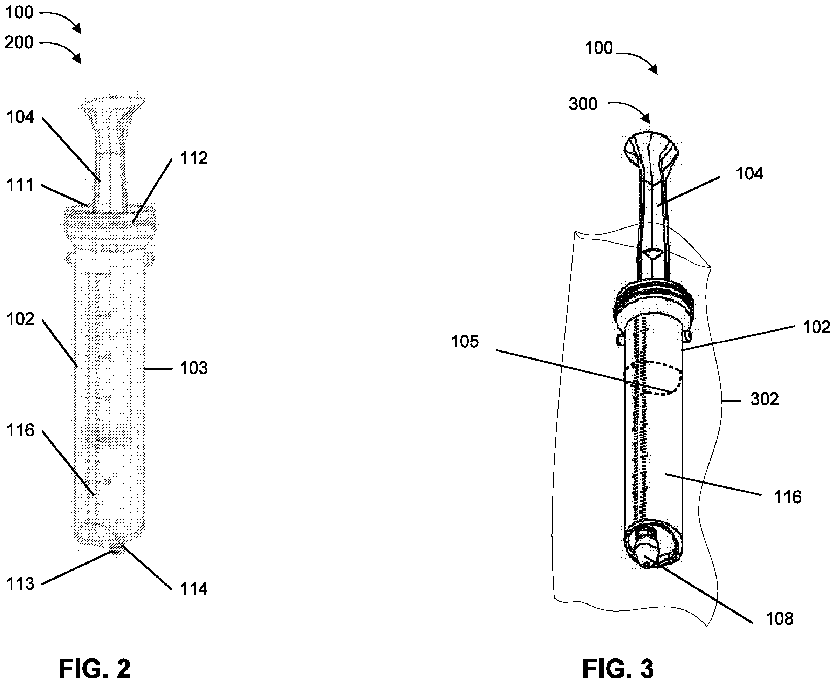

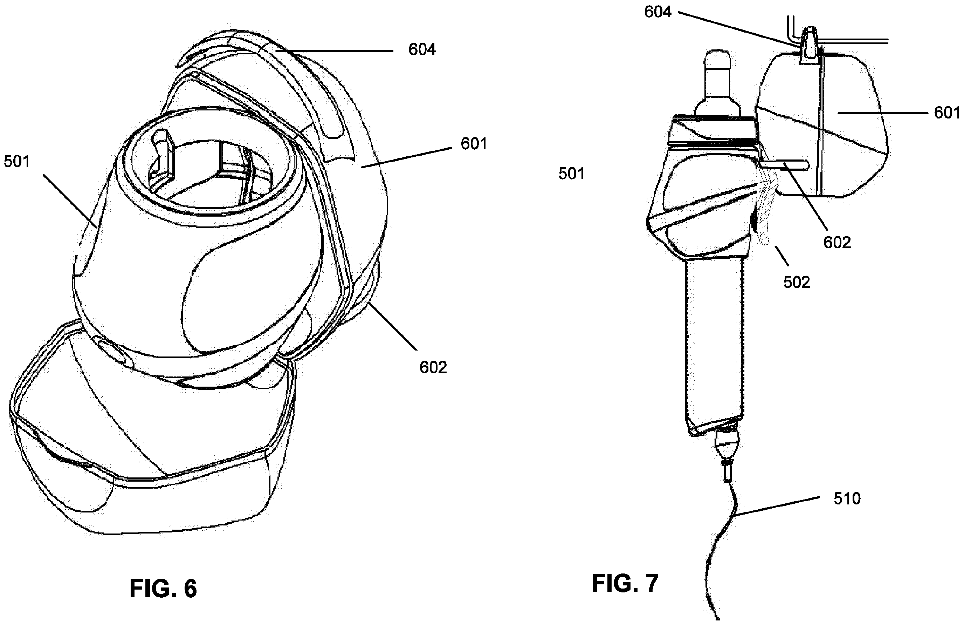

Body 102 includes a tube portion 103 featuring an interior cavity 116 for holding a liquid. At one end of tube portion 103, body 102 includes a wide opening 111 with external screw threads 112. At the opposite end of tube portion 103, body 102 includes a narrow opening 113 with external screw threads 114. Narrow opening 113 has a diameter which is smaller than the diameter of tube portion 103, and wide opening 111 has a diameter substantially equal to or greater than the diameter of tube portion 103. Body 102 also includes a scale mark or graduation mark 130 printed or etched onto container 100 for measuring an amount of liquid inside container 100. Scale 130 is written in a direction that makes it easy to read while narrow opening 113 is pointed downwards and wide opening 111 is pointed upwards as is further demonstrated in Figures 2, 5, and 7. A second scale mark or graduation mark 132 is printed or etched onto container 100 in the opposite direction of scale 130, so that it is easy to read while wide opening 111 is pointed downwards and narrow opening 113 is pointed upwards, as in Figure 4. Plunger 104 is sized so that it can sealingly and slidably fit inside cavity 116 of body 102, wherein a seal 105 of plunger 104 is fitted to slide along the internal walls of tube portion 103. Plunger 104 can be inserted into tube portion 103 through wide opening 111 with its seal 105 proximate to narrow opening 113, allowing the sucking up of liquid through narrow opening 113, as a typical syringe is operated. Nipple 106 includes internal screw threads 118 that correspond to external screw threads 112 on body 102. Cap 108 and feeding tube 110 each include internal screw threads 120 and 122, respectively, that correspond to external screw threads 114 on body 102. In place of corresponding screw threads 112, 114, 118, 120 and 122 other corresponding complementary attachment elements, arrangements or configurations may be provided on the respective parts of container 100, such as corresponding snap fit or mounting rail and track elements. Optional gripping sleeve 501 has two open ends and is configured to selectively embrace container 100 by sliding externally onto and off of body 102 of container 100. Gripping sleeve 501 has widened external dimensions configured to facilitate holding of container body 102 in feed bottle mode 400. Gripping sleeve 501 has an optional clip 502 or other securing element, for securing to an object, such as an article of clothing of the feeder (mother or nurse), or hanging by further means, such as those described below with reference to carrier 601 and to Figure 6, which is a perspective view of the sleeve and carrier of Figure 1. Carrier 601 is a case or a box configured to open and close and has an interior fitted to encapsulate gripping sleeve 501. Carrier 601 may be opened to allow Gripping sleeve 501 to be placed inside and then closed again to store Gripping sleeve 501 inside (see Figure 6). Carrier 601 also has a handle 602 and a hanger 604. Handle 602 is configured to be attached to clip 502 of gripping sleeve 501 and provide hanging suspension thereto (see Figure 7). Hanger 604 is configured to be hung on an object, such as a hook or a suspension rack (which are often already nearby) (see Figure 7). Carrier 601 may be opened to remove gripping sleeve 501 and then closed again to be attached to gripping sleeve 501, using the clip 502 and handle 602, and used to help hang container 100 from an object, such as a piece of medical or other structural equipment, hook or suspension rack (see Figures 6 and 7).

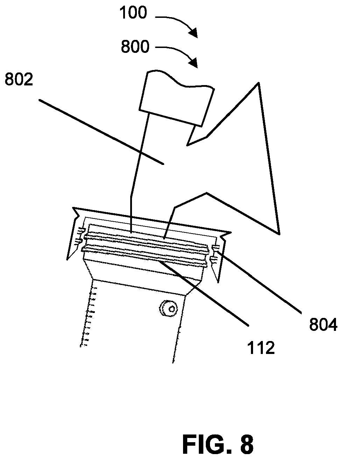

An optional breast pump 802 (see Figure 8) with internal screw threads 804 that correspond to external screw threads 112 on body 102 may be provided. Breast pump 802 is configured for pumping milk from a breast into the container body 102, as a typical breast pump is operated. Breast pump 802 may be provided together with container 100 as part of the kit, or it may be any breast pump that container 100 is configured to attach to.

Container 100 has a plurality of modes or configurations: a syringe mode, a storage mode, a feed-bottle mode, a feed-tube mode, and optionally a pump vessel mode.

Reference is now made to Figure 2, which is a perspective view of the container 100 of Figure 1, in a syringe mode 200, constructed and operative in accordance with an embodiment of the present invention. In syringe mode 200, plunger 104 is sealingly and slidably located in cavity 116 of tube portion 103 of body 102, so that it seals one end of container 100 adjacent wide opening 111 and is capable of creating a vacuum in cavity 116 when pulled towards that end. In syringe mode 200 container 100 is capable of sucking up a liquid by inserting narrow opening 113 of the container 100 in a liquid and pulling out plunger 104 (pulling plunger 104 away from narrow opening 113 and towards wide opening 111). Syringe mode 200 also provides for application of the milk contained in body 102 through narrow opening 113, by pushing plunger 104, for direct feeding of the baby (or for a further vessel if a simple syringe functioning of the multi-functional kit 100 satisfies the user).

Reference is now made to Figure 3, which is a perspective view of the container 100 of Figure 1, in a storage mode 300, constructed and operative in accordance with an embodiment of the present invention. Plunger 104 remains inserted (perforated line denotes the position of plunger seal 105) and the narrow opening 113 is sealed by cap 108 thereby sealingly enclosing tube portion 103 so that liquid contained therein (within enclosed cavity 116) can be safely stored. An optional plastic bag wrapper 302 with an ID or quick response (QR) barcode can be used to help store and identify container 100.

Figure 4 is a perspective view of the container 100 of Figure 1 in a feed-bottle mode 400, constructed and operative in accordance with an embodiment of the present invention. In feed-bottle mode 400 nipple 106 is connected to body 102 at wide opening 111 using corresponding screw threads 112 and 118. Cap 108 is connected to body 102 at narrow opening 113 using corresponding screw threads 120 and 114. In feed-bottle mode 400, container 100 is closed at both ends 111 and 113 so that a liquid in container 100 is released only by sucking on nipple 106 when nipple 106 is used for feeding (nipple 106 is pointed downwards directly or at an angled posture so that liquid that settles downwards can pour from nipple 106). Optional gripping sleeve 501 is slid externally around tube portion 103 of container 100 allowing comfortable gripping in a feeding posture. Clip 502 can be used to hold container 100 in an upright manner (with nipple 106 facing upwards) when attached to an article of clothing (such as a shirt collar) in between bottle feedings. This makes container 100 easy to carry from place to place, and helps prevents a user from misplacing container 100.

Reference is now made to Figures 5 and 7. Figure 5 is a perspective view of the container 100 of Figure 1 in a feed-tube mode 500, constructed and operative in accordance with an embodiment of the present invention. Figure 7 is a perspective view of the container of Figure 1 in a feed-tube mode and being held by the sleeve and carrier. In feed-tube mode 500 nipple 106 is connected to body 102 at wide opening 111 using corresponding screw threads 112 and 118. Feeding tube 110 is connected to body 102 at narrow opening 113 using corresponding screw threads 122 and 114. Tube portion 510 of feeding tube 100 hangs therefrom towards the fed infant. In feed-tube mode 500 container 100 is capable of expressing a liquid through feeding tube 110 by pumping or depressing nipple 106 (pushing nipple 106 towards narrow opening 113). Optional gripping sleeve 501 is preferably slid externally around tube portion 103 of container 100 so that clip 502 can be attached to any hook or rack, or to handle 602 of carrier 601 and used to hold container 100 in an upright manner (with nipple 106 facing upwards) when attached to a piece of structural equipment using hanger 604, especially during tube feedings, as best seen in Figure 7. Hanger 604 makes container 100 easy to hang from a corresponding hook (Figure 7).

Reference is now made to Figure 8, which is a perspective view of the container 100 of Figure 1, in a pump vessel mode 800, constructed and operative in accordance with an embodiment of the present invention. In pump vessel mode 800 a breast pump 802 is connected to body 102 at wide opening 111 using corresponding screw threads 112 and 804. Cap 108 is connected to body 102 at narrow opening 113 using corresponding screw threads 120 and 114. In pump vessel mode 800 milk may be pumped from a breast directly into the container body 102, thereby eliminating the transfer of milk from another vessel.

The operation of container 100 will now be further discussed. Reference is now made to Figures 1 to 5, and 8. After milk has been expressed or pumped by a mother into an external vessel (not shown), container 100 is assembled or configured into syringe mode 200 by placing plunger 104 into body 102 and used to suck up and measure an amount of milk for feeding to the baby by inserting narrow opening 113 into the milk and pulling plunger 104 towards wide opening 111 (Figure 2). Container 100 is now converted into storage mode 300 by connecting cap 108 over narrow opening 113 and used to store the milk until feeding time (Figure 3). If no storage is required, container 100 can also be converted from syringe mode 200 directly into feed-bottle mode 400, or feed-tube mode 500, as desired. When it is feeding time, container 100 is then converted, either from syringe mode 200 or storage mode 300, into feed-bottle mode 400 by pulling plunger 104 through wide opening 111 until its entire removal from body 102 and connecting nipple 106 over wide opening 111 (Figure 4). Optionally, gripping sleeve 501 is sled over container 100 to an embracing position there over, providing a comfortable grip for a feed-bottle posture (Figure 4) or a clasp. After feeding milk to the baby using feed-bottle mode 400, if any milk remains to be fed to the baby, or in case the baby could not be fed by using feed-bottle mode, the container 100 is converted into feed-tube mode 500 by removing cap 108 and replacing it with feeding tube 110 (Figures 5 and 8). Optionally, gripping sleeve 501 is slid over container 100 to an embracing position there over, providing a clip 502 for temporary clipping or hanging of container 100 in between continuous feeding sessions. Feed-tube mode 500 can be also selected directly after milk is inserted into container 100 at the syringe mode 200 or at the pump vessel mode 800. In feed-tube mode 500, pumping or depressing nipple 106 (or pushing nipple 106 towards narrow opening 113) will pressurize air and liquids enclosed within cavity 116 and cause milk in body 102 to be expressed from the container 100 through feeding tube 110.

Alternatively, container 100 may initially be configured in pump vessel mode 800 by connecting breast pump 802 over wide opening 111 and cap 108 over narrow opening 113 and used to pump milk from a breast into the container body102 by placing breast pump 802 on a breast. Container 100 can then be converted from pump vessel mode 800 into any of the other modes (e.g., storage mode 300, feed-bottle mode 400, or feed-tube mode 500) as desired, in the manner described in detail above.

The container of the present invention simplifies the process of feeding a preemie by reducing the number of vessels through which the milk is transferred before it is delivered to the baby. This reduction in amount of transfers helps preserve the quality of the milk and its essential components by reducing the milk's exposure to possible infection or contamination, and reducing the amount of milk and milk components loss due to transfer between vessels.

The various elements of the container may be provided as a baby feeding kit.

While certain embodiments of the disclosed subject matter have been described, so as to enable one of skill in the art to practice the present invention, the preceding description is intended to be exemplary only. It should not be used to limit the scope of the disclosed subject matter, which should be determined by reference to the following claims.

## Claims

1. A baby feeding kit,
wherein the kit is operational in syringe mode, storage mode, feed-bottle mode, and feed-tube mode,
the kit comprising:
a container body configured for holding a liquid;
said container body comprising:
a tube portion (103) defining a cavity therein;
a wide opening (111) disposed at one end of said tube portion;
and
a narrow opening (113) disposed at an opposite end of said tube portion;
a plunger (104) sized to removably and sealingly fitting inside said cavity and slidably move there along when said container is in a syringe mode, and sealing said tube portion when said container is in a storage mode;
an attachable nipple (106) operational for sealingly attaching to said wide opening, configured for releasing a liquid held in the container body when sucked on when said container is in a feed-bottle mode in which said plunger is removed, and for pressurizing fluids when said container is in a feed-tube mode, and which is removed when said container is in a syringe or storage mode;
an attachable cap (108) operational for sealingly attaching to said narrow opening, configured to seal said narrow opening of the container when in storage or in feed-bottle mode; and
a feeding tube (110) operational for sealingly attaching to said narrow opening, configured for releasing a liquid held in the container body when said container is in a feed-tube mode.

2. The baby feeding kit of claim 1, wherein at least one of said wide opening and said narrow opening comprises an attachment element that corresponds to attaching to a complementary attaching element disposed on at least one of: said nipple, said cap, and said feeding tube.

3. The baby feeding kit of claim 2, wherein said attachment element and said complementary attachment element comprise screw threads.

4. The baby feeding kit of claim 1, further comprising a gripping sleeve configured to slide externally onto and off of said container body, and to provide a grip support facilitating holding of said container body in said feed bottle mode.

5. The gripping sleeve of claim 4, comprising a securing element for securing said gripping sleeve to an object.

6. The baby feeding kit of claim 5, wherein said securing element comprises a clip mounted on said gripping sleeve;

7. The baby feeding kit of claim 4, further comprising a carrier for removably storing and carrying said gripping sleeve.

8. The carrier of claim 7, further comprising a handle for providing a hanging suspension for said gripping sleeve; and a hanger for hanging said carrier.

9. The baby feeding kit of claim 1, wherein said kit is operational in a pump vessel mode, wherein said wide opening is configured for attaching to a breast pump for pumping milk from a breast into said container body when said container is in pump vessel mode.

10. The baby feeding kit of claim 1, wherein said kit is operational in a pump vessel mode, wherein said kit further comprises a breast pump operational for attaching to said wide opening, configured for pumping milk from a breast into said container body when said container is in a pump vessel mode.

## Patentansprüche

1. Babyfütter-Kit,
wobei das Kit in einem Spritzenmodus, Speichermodus, Fütterflaschenmodus und Fütterleitungsmodus betriebsbereit ist,
wobei das Kit Folgendes umfasst:
einen Behälterkörper, der zum Halten einer Flüssigkeit konfiguriert ist; wobei der Behälterkörper Folgendes umfasst:
einen Leitungsabschnitt (103), der einen Hohlraum darin definiert;
eine breite Öffnung (111), die an einem Ende des Leitungsabschnitts angeordnet ist;
und
eine schmale Öffnung (113), die an einem gegenüberliegenden Ende des Leitungsabschnitts angeordnet ist;
einen Kolben (104), der bemessen ist, um abnehmbar und abdichtend in den Hohlraum zu passen und sich dort entlang gleitend bewegt, wenn der Behälter in einem Spritzenmodus ist, und den Leitungsabschnitt abdichtet, wenn der Behälter in einem Speichermodus ist;
einen anbringbaren Nippel (106), der zum abdichtenden Anbringen an der breiten Öffnung betriebsbereit ist, der konfiguriert ist, um eine Flüssigkeit freizugeben, die in dem Behälterkörper gehalten wird, wenn sie angesaugt wird, wenn der Behälter in einem Fütterflaschenmodus ist, in dem der Kolben entfernt wird, und um Flüssigkeiten unter Druck zu setzen, wenn der Behälter in einem Fütterleitungsmodus ist, und der entfernt wird, wenn der Behälter in einem Spritzen- oder Speichermodus ist;
eine anbringbare Kappe (108), die zum abdichtenden Anbringen an der schmalen Öffnung funktionsfähig ist, die konfiguriert ist, um die schmale Öffnung des Behälters abzudichten, wenn sie in dem Speicher- oder Fütterflaschenmodus ist; und
eine Fütterleitung (110), die zum abdichtenden Anbringen an der schmalen Öffnung betriebsbereit ist, die konfiguriert ist, um eine in dem Behälterkörper gehaltene Flüssigkeit freizugeben, wenn der Behälter in einem Fütterleitungsmodus ist.

2. Babyfütter-Kit nach Anspruch 1, wobei mindestens eine der breiten Öffnung und der schmalen Öffnung ein Anbringungselement umfasst, das einem Anbringen an einem komplementären Anbringungselement entspricht, das an mindestens einem der folgenden angeordnet ist: dem Nippel, der Kappe und der Fütterleitung.

3. Babyfütter-Kit nach Anspruch 2, wobei das Befestigungselement und das ergänzende Befestigungselement Schraubengewinde umfassen.

4. Babyfütter-Kit nach Anspruch 1, ferner umfassend eine Greifhülse, die konfiguriert ist, um außen auf und von dem Behälterkörper zu gleiten und eine Griffstütze bereitzustellen, die ein Halten des Behälterkörpers in dem Fütterflaschenmodus erleichtert.

5. Greifhülse nach Anspruch 4, umfassend ein Sicherungselement zum Befestigen der Greifhülse an einem Gegenstand.

6. Babyfütter-Kit nach Anspruch 5, wobei das Befestigungselement einen Clip umfasst, der an der Greifhülse montiert ist.

7. Babyfütter-Kit nach Anspruch 4, ferner umfassend einen Träger zum abnehmbaren Aufbewahren und Tragen der Greifhülse.

8. Träger nach Anspruch 7, ferner umfassend einen Griff zum Bereitstellen einer Aufhängung für die Greifhülse; und einen Aufhänger zum Aufhängen des Trägers.

9. Babyfütter-Kit nach Anspruch 1, wobei das Kit in einem Pumpgefäßmodus betriebsbereit ist, wobei die breite Öffnung zum Anbringen an einer Milchpumpe zum Pumpen von Milch von einer Brust in den Behälterkörper konfiguriert ist, wenn der Behälter im Pumpgefäßmodus ist.

10. Babyfütter-Kit nach Anspruch 1, wobei das Kit in einem Pumpgefäßmodus betriebsbereit ist, wobei das Kit ferner eine Milchpumpe umfasst, die zum Anbringen an der breiten Öffnung betriebsbereit ist, die konfiguriert ist, um Milch von einer Brust in den Behälterkörper zu pumpen, wenn der Behälter in einem Pumpgefäßmodus ist.

## Revendications

1. Kit d'alimentation pour bébé,
le kit étant opérationnel en mode seringue, en mode stockage, en mode biberon et en mode sonde gastrique,
le kit comprenant :
un corps de contenant conçu pour contenir un liquide ;
ledit corps de contenant comprenant :
une partie de tube (103) définissant une cavité dans celle-ci ;
une ouverture large (111)
disposée à une extrémité de ladite partie de tube ;
et
une ouverture étroite (113)
disposée à une extrémité opposée de ladite partie de tube ;
un piston (104)
dimensionné pour s'adapter de façon amovible et étanche à l'intérieur de ladite cavité et se déplacer de manière coulissante le long de celle-ci lorsque ledit contenant est en mode seringue et scellant ladite partie de tube lorsque ledit contenant est en mode stockage ;
une tétine fixable (106)
opérationnelle pour être fixée de manière étanche à ladite ouverture large, conçue pour libérer un liquide contenu dans le corps de contenant lorsqu'elle est tétée lorsque ledit contenant est dans un mode biberon dans lequel ledit piston est retiré, et pour mettre sous pression des fluides lorsque ledit contenant est dans un mode sonde gastrique, et qui est retirée lorsque ledit contenant est dans un mode seringue ou stockage ;
un capuchon fixable (108)
opérationnel pour se fixer de façon étanche à ladite ouverture étroite, conçu pour sceller ladite ouverture étroite du contenant lorsqu'il est en mode stockage ou biberon ; et
une sonde gastrique (110)
opérationnelle pour se fixer de façon étanche à ladite ouverture étroite, conçue pour libérer un liquide contenant dans le corps de contenant lorsque ledit contenant est dans un mode sonde gastrique.

2. Kit d'alimentation pour bébé de la revendication 1, dans lequel au moins l'une de ladite ouverture large et de ladite ouverture étroite comprend un élément de fixation qui correspond à une fixation à un élément de fixation complémentaire disposé que au un élément parmi : ladite tétine, ledit capuchon et ladite sonde gastrique.

3. Kit d'alimentation pour bébé de la revendication 2, dans lequel ledit élément de fixation et ledit élément de fixation complémentaire comprennent des filets de vis.

4. Kit d'alimentation pour bébé de la revendication 1, comprenant en outre un manchon de préhension conçu pour coulisser de façon externe sur ledit corps de contenant et s'enlever de celui-ci, et pour fournir un support de préhension facilitant la prise dudit corps de contenant dans ledit mode biberon.

5. Manchon de préhension de la revendication 4, comprenant un élément d'arrimage destiné à arrimer ledit manchon de préhension à un objet.

6. Kit d'alimentation pour bébé de la revendication 5, dans lequel ledit élément d'arrimage comprend une attache montée sur ledit manchon de préhension.

7. Kit d'alimentation pour bébé de la revendication 4, comprenant en outre un support destiné à stocker de manière amovible et transporter ledit manchon de préhension.

8. Support de la revendication 7, comprenant en outre un manche destiné à fournir une suspension de mise en suspension pour ledit manchon de préhension ; et un élément de suspension pour suspendre ledit support.

9. Kit d'alimentation pour bébé de la revendication 1, ledit kit étant opérationnel dans un mode récipient à pompe, dans lequel ladite ouverture large est conçue pour se fixer à un tire-lait afin de pomper du lait d'un sein audit corps de contenant lorsque ledit contenant est en mode récipient à pompe.

10. Kit d'alimentation pour bébé de la revendication 1, ledit kit étant opérationnel dans un mode récipient à pompe, ledit kit comprenant en outre un tire-lait opérationnel pour une fixation à ladite ouverture large, conçu pour pomper du lait d'un sein audit corps de contenant lorsque ledit contenant est dans un mode récipient à pompe.
